# EUROPEAN PATENT APPLICATION

(11) **EP 4 360 579 A1**
(43) Date of publication of application: **01.05.2024**
(21) Application number: 23204755.5
(22) Date of filing: 19.10.2023
(51) Int. Cl.: A61B 34/00, A61B 1/00

(54) **MAGNETIC ACTUATION DEVICE USING PERMANENT MAGNETS AND SYSTEM USING THE SAME**

(30) Priority: 26.10.2022 KR 20220138860
(71) Applicant: Daegu Gyeongbuk Institute Of Science and Technology, Daegu 42988 (KR)
(72) Inventor: CHOI, Hong Soo, 42951 Daegu (KR); LEE, Hak Joon, 43014 Daegu (KR); KIM, Jin Young, 43016 Daegu (KR); ABBASI, Sarmad Ahmad, 42988 Daegu (KR); AWAIS, Ahmed, 42988 Daegu (KR); CHOWDHURY, A M Masum Bulbul, 43014 Daegu (KR); LATIFI, Gharamaleki Nader, 42988 Daegu (KR)
(74) Representative: Stolmár & Partner Patentanwälte PartG mbB

(57) **Abstract**

Provided are a magnetic actuation device using a permanent magnet which may generate a magnetic field for a long time with lower power and lower heat by using the permanent magnet, and a system using the same. The magnetic actuation device may utilize the plurality of permanent magnets for controlling an alignment direction with two degrees of freedom to thus exclude an actuation element limiting space utilization, such as a robot arm, thus allowing an operator using a medical device to perform a safe procedure on a patient.

## Description

### CROSS-REFERENCE TO RELATED APPLICATIONS

This application claims priority under 35 U.S.C. §119 to Korean Patent Application No. 10-2022-0138860, filed on Oct 26, 2022, in the Korean Intellectual Property Office, the disclosure of which is incorporated herein by reference in its entirety.

### TECHNICAL FIELD

The following disclosure relates to a magnetic actuation device using permanent magnets and a system using the same.

### BACKGROUND

In recent years, a medical device using a microrobot and an electromagnetic device has been used in a medical field. Such a small microrobot may be inserted into a human body and require fine adjustment from the outside. To this end, the electromagnetic device which may finely adjust and actuate the microrobot or the like may be used in the field.

Conventionally, the electromagnetic device used as the medical device may use an electromagnetic coil. The electromagnetic coil may consume a lot of power and generate a lot of heat. For this reason, it is difficult to actuate the electromagnetic device using the electromagnetic coil for a long time.

In addition, a mechanical movement of the electromagnetic coil operated in real time may greatly restrict an operator's approach to a patient, and poor compatibility of the electromagnetic coil with a medical imaging system such as an X-ray system may also result in a very limited available angle for capturing an image.

### [Related Art Document]

### [Patent Document]

(Patent Document) Japanese Patent Laid-Open Publication No. 2021-522960 (published on September 2, 2021)

### SUMMARY

An embodiment of the present disclosure is directed to providing a magnetic actuation device using permanent magnets with lower power consumption, lower heat, and a higher degree of freedom for easy space utilization by using the permanent magnets to generate a magnetic field, and a system using the same.

In one general aspect, provided is a magnetic actuation device including: a base; and at least two magnetic field generation modules each disposed on the base and including a permanent magnet, the permanent magnet being rotated about a horizontal axis parallel to the base or a vertical axis perpendicular to the base.

In another general aspect, provided is a magnetic actuation system which includes the magnetic actuation device as described above, the magnetic actuation system including: a bed; the magnetic actuation device installed in the bed; an imaging device connected to the magnetic actuation device; and a control device controlling the magnetic actuation device and the imaging device.

### BRIEF DESCRIPTION OF THE DRAWINGS

FIG. 1 is a view showing a magnetic actuation device according to an embodiment of the present disclosure.
FIG. 2 is a view showing a magnetic field generation module of FIG. 1.
FIG. 3 is a view showing a fixing unit of the magnetic field generation module of FIG. 2.
FIGS. 4A to 4E are views showing various shapes and arrangements of permanent magnets used in the embodiments of the present disclosure.
FIGS. 5A and 5B are views each showing that an actuation unit is coupled to the magnetic field generation module of FIG. 2.
FIG. 6 is a view showing rotation of the permanent magnet of FIG. 2 in two axial directions.
FIG. 7 is a view showing a magnetic generation module of a magnetic actuation device in another example of the present disclosure.
FIG. 8 is a view showing that an actuation unit is coupled to the magnetic field generation module of FIG. 7.
FIG. 9 is a view showing rotation of a permanent magnet of in FIG. 7 in two axial directions.
FIG. 10 is a view showing a magnetic actuation system according to another embodiment of the present disclosure.
FIG. 11 is a view showing a sensor unit included in the magnetic field generation module according to the embodiments of the present disclosure.
FIG. 12 is a view showing a result acquired by simulating the direction and strength of a magnetic field generated by an electromagnetic coil for controlling alignment of the permanent magnets in the two axial directions.
FIGS. 13A and 13B are views each showing a result acquired by simulating the direction and strength of the magnetic field generated by aligning the plurality of permanent magnets in the two axial directions.
FIG. 14 is a view showing a result acquired by applying the magnetic generation module according to the embodiments of the present disclosure to the magnetic actuation system according to another embodiment of the present disclosure and simulating the direction and strength of the magnetic field generated by aligning the plurality of permanent magnets in the two axial directions.

### DETAILED DESCRIPTION OF EMBODIMENTS

Hereinafter, specific embodiments of the present disclosure are described with reference to the drawings.

Further, in describing the embodiments of the present disclosure, omitted is a detailed description of a case where it is decided that the detailed description of the known function or configuration related to the present disclosure may unnecessarily obscure the gist of the present disclosure.

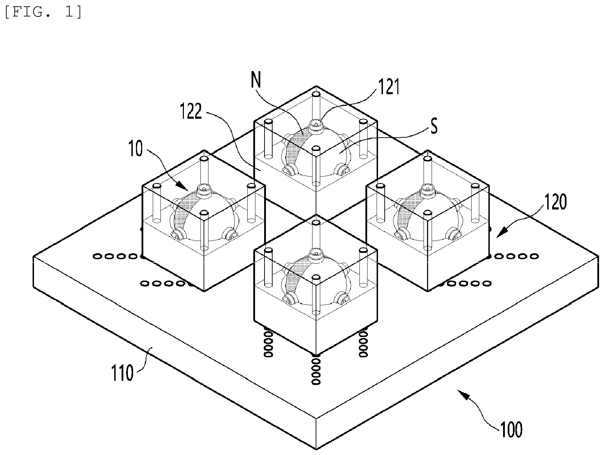

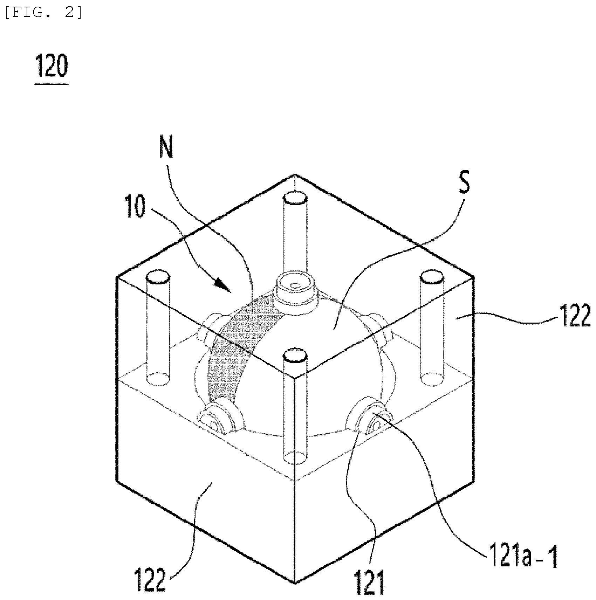

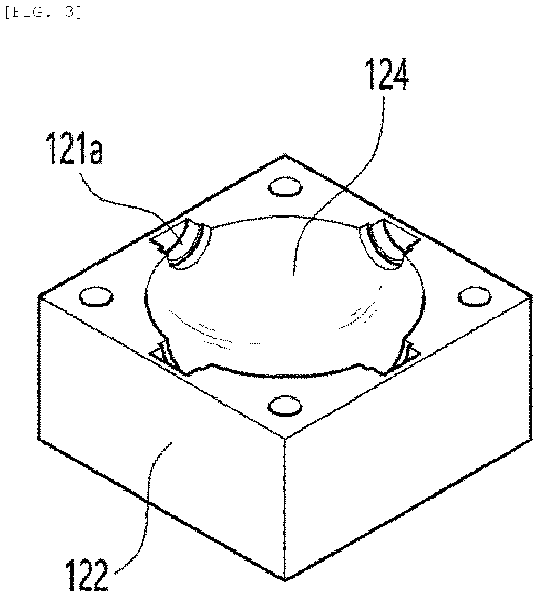

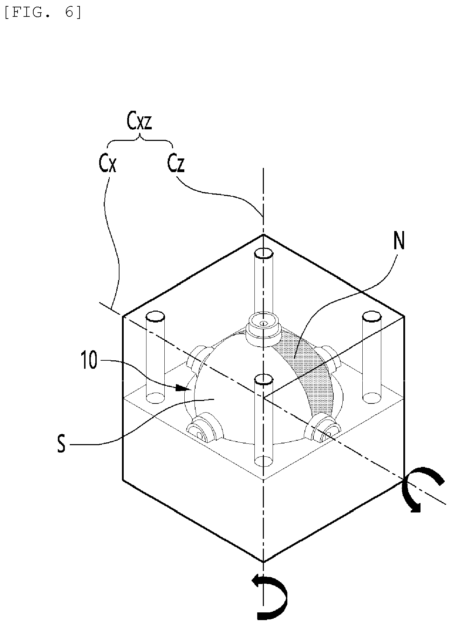

Referring to FIGS. 1 to 6, a magnetic actuation device 100 according to an embodiment of the present disclosure may include a base 110 and a magnetic field generation module 120.

The magnetic field generation module 120 may be disposed on the base 110. At least two magnetic field generation modules 120 may be disposed on the base 110. The magnetic actuation device 100 according to an embodiment of the present disclosure may be provided for distributing a magnetic field in a specific direction through alignment of the permanent magnets 10. At least two or more or at least three or more magnetic field generation modules 120 including the permanent magnets 10 may be disposed.

The magnetic field generation module 120 may include the permanent magnet 10. (In the drawings, the N pole of the permanent magnet 10 is black and the N pole of the permanent magnet 10 is white.) The permanent magnet 10 may be included in the magnetic field generation module 120, and rotated about a horizontal axis Cx parallel to the base 110 or a vertical axis Cz perpendicular to the base 110.

The base 110 may be disposed to be parallel to the ground. The permanent magnet 10 may be rotated about the horizontal axis Cx parallel to the base 110 with respect to the base 110 or the vertical axis Cz perpendicular to the base 110.

The magnetic field generation module 120 may include a rotation unit 121 rotating the permanent magnet 10 in two axial directions of the horizontal axis Cx or the vertical axis Cz. The rotation unit 121 may be fixed to a fixing unit 122. The rotation unit 121 may receive a rotational force by an actuation unit 123.

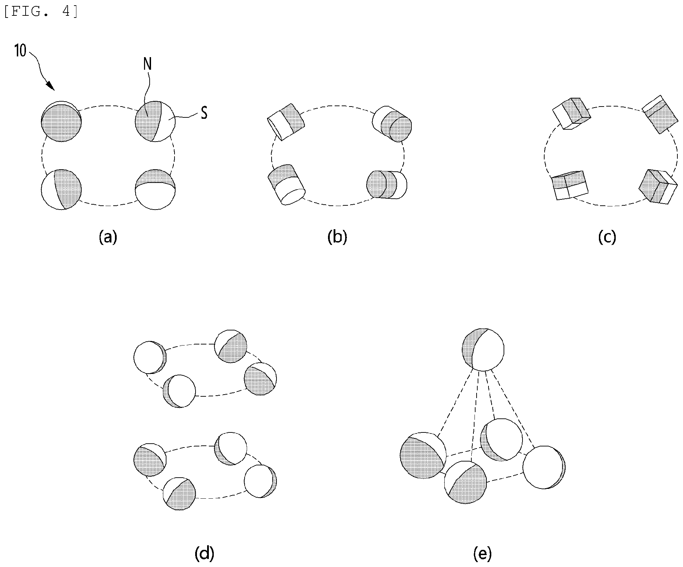

The fixing unit 122 may have an accommodation space 124 for accommodating the permanent magnet 10. The fixing unit 122 may be divided into two pieces. Both the pieces may form the accommodation space 124 corresponding to a shape of the permanent magnet 10. The accommodation space 124 formed by both the pieces may correspond to the shape of the permanent magnet 10. The permanent magnet 10 may have various shapes as shown in FIGS. 4A to 4E. The permanent magnet 10 applied to the magnetic field generation module 120 according to an embodiment of the present disclosure may have a spherical shape as shown in FIG. 4A. The reason is that the permanent magnet 10 needs to be rotated in the direction of the horizontal axis Cx or that of the vertical axis Cz while being accommodated in the accommodation space 124 of the fixing unit 122.

The fixing unit 122 may include the rotation unit 121 guiding the rotation of the permanent magnet 10 that is disposed in a surface of the fixing unit 122 in contact with the permanent magnet 10 accommodated in the accommodation space 124. The rotation unit 121 may be formed as each rotation groove 121a in the front, rear, left, right, upper and lower surfaces of the rotation unit 121 in contact with the permanent magnet 10 accommodated in the accommodation space 124 of the fixing unit 122. A guide ball 121a-1 may be disposed in each rotation groove 121a and guide the permanent magnet 10 to be rotated in the accommodation space 124.

The actuation unit 123 may be an electromagnetic coil 123a controlling a rotation direction of the permanent magnet 10 based on a current, and may be disposed on an outer surface of the rotation unit 121.

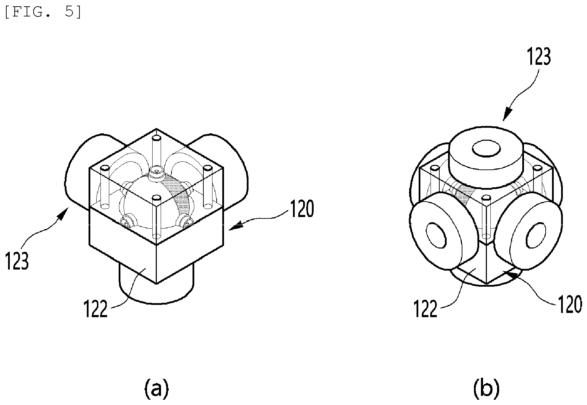

The actuation unit 123 may be installed on each of the three surfaces of the fixing unit 122 as shown in FIG. 5A. Alternatively, the actuation unit 123 may be installed on each of the six surfaces of the fixing unit 122 as shown in FIG. 5B. In an embodiment of the present disclosure, the fixing unit 122 may have a cube shape, and the actuation unit 123, that is, the electromagnetic coil 123a, may be selectively installed on a surface of the fixing unit 122 in the cube shape or installed on all the surfaces of the fixing unit 122 in the cube shape. Here, the permanent magnets 10 may be more finely aligned as the more electromagnetic coils 123a are installed.

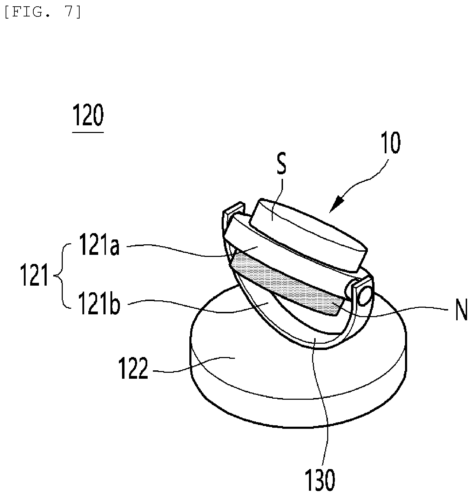

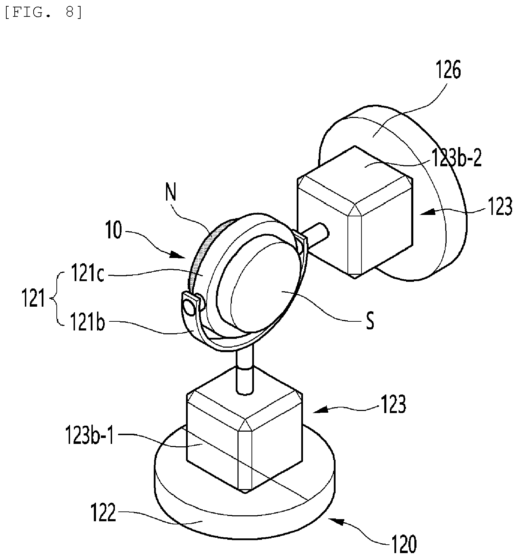

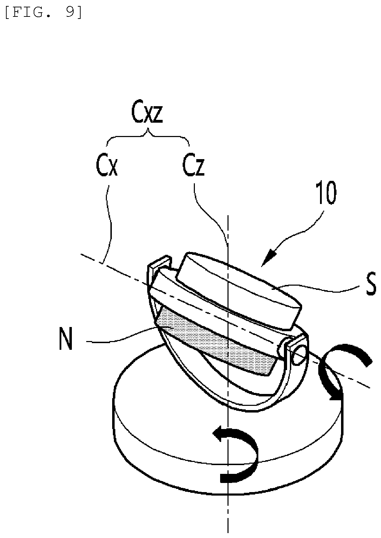

The description describes the magnetic actuation device 100 in another example of the present disclosure with further reference to FIGS. 7 to 9. Components of the magnetic actuation device 100 that are not described herein are the same as those of the magnetic actuation device 100 according to an embodiment of the present disclosure.

The magnetic actuation device 100 in another example of the present disclosure has a difference in the magnetic field generation module 120.

The rotation unit 121 of the magnetic field generation module 120 may be installed on the fixing unit 122. The rotation unit 121 may include a first rotation ring 121b rotating the permanent magnet 10 about the vertical axis Cz and a second rotation ring 121c installed on the first rotation ring 121b and rotating the permanent magnet 10 about the horizontal axis Cx.

The first rotation ring 121b may be installed on the fixing unit 122 fixed to the base 110, and the second rotation ring 121c may be installed at the first rotation ring 121b.

The first rotation ring 121b may have a semicircular arc shape. In the semicircular arc, the central portion of the arc may be coupled to the fixing unit 122 by a rotation coupling unit 130. The first rotation ring 121b may be rotated about the vertical axis Cz by the rotation coupling unit 130. The rotation coupling unit 130 may use a rotary joint.

The second rotation ring 121c may be coupled to both ends of an opening of the semicircular arc of the first rotary ring 121b by the rotation coupling unit 130. The second rotation ring 121c may have a circular shape. The second rotation ring 121c may fix the permanent magnet 10 while surrounding the outside of the permanent magnet 10. Here, the permanent magnet 10 may have a spherical shape shown in FIG. 4A or a cylindrical shape shown in FIG. 4B.

The second rotation ring 121c may be rotatably coupled to the first rotation ring 121b by the rotation coupling unit 130 and rotated about the horizontal axis Cx.

The actuation unit 123 may be an actuator 123b, and may be connected to each of the first rotation ring 121b and the second rotation ring 121c. A first actuator 123b-1 may be connected to the first rotation ring 121b, and a second actuator 123b-2 may be connected to the second rotation ring 121c. The first actuator 123b-1 connected to the first rotation ring 121b may transmit a rotational force to the vertical axis Cz. The second actuator 123b-2 connected to the second rotation ring 121c may transmit a rotational force to the horizontal axis Cx.

The second rotation ring 121c may be connected to the first rotation ring 121b and rotated about the vertical axis Cz together with the first rotation ring 121b by the rotation of the first rotation ring 121b. The second actuator 123b-2 connected to the second rotation ring 121c may be connected to a side guide 126 corresponding to its rotation about the vertical axis Cz. The side guide may guide the second actuator 123b-2 to be rotated about the vertical axis Cz.

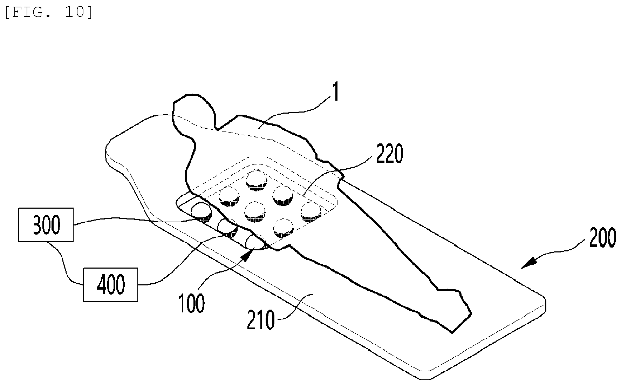

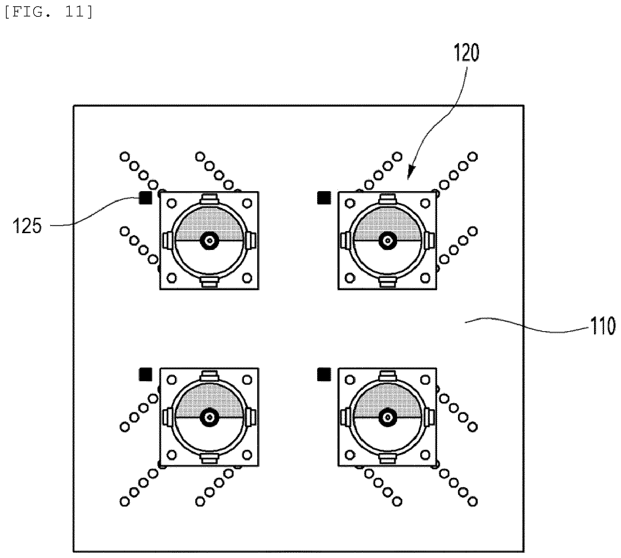

The description describes a magnetic actuation system 200 including the magnetic actuation device 100 in each example of the present disclosure with further reference to FIGS. 10 and 11. The magnetic actuation system 200 may use the same magnetic actuation device 100 as that in each of the above-described examples of the present disclosure.

The magnetic actuation system 200 accordinfg to another embodiment of the present disclosure may include a bed 210, the magnetic actuation device 100, an imaging device 300, and a control device 400.

The bed 210 may be provided for a patient 1 to lie down or sit down to be treated or examined with a magnetic robot or the like using the magnetic actuation device 100.

The magnetic actuation device 100 may be installed in the bed 210, and may be installed in a rear surface of the bed 210 to avoid interference with the imaging device 300 or the like. Here, a position of the rear surface where the magnetic actuation device 100 is installed may be changed based on the body of the patient 1. For example, the magnetic actuation device 100 may be positioned in a middle portion of the bed 210 in a length direction when measuring the heart of the patient 1 as shown in FIG. 10. The magnetic actuation device 100 may be positioned in an upper portion of the bed 210 in the length direction when measuring the head of the patient 1. The magnetic actuation device 100 may be selectively installed in the bed 210 based on a measurement position of the patient 1. The permanent magnet 10 may be disposed in a flat shape as shown in FIGS. 4A to 4C when the magnetic actuation device 100 is installed on the rear surface of the bed 210.

Alternatively, the magnetic actuation device 100 may be disposed in a support part (not shown) separately provided at the bed 210. The support part may be provided for installing the magnetic actuation device 100 outside the bed 210 instead of inside the bed 210, and may have a frame shape. Here, the permanent magnets 10 may be disposed in a three-dimensional shape as shown in FIG. 4D or 4E.

The imaging device 300 may be connected to the magnetic actuation device 100. The imaging device 300 may be provided for confirming a measurement process or a treatment process in real time like a display.

The control device 400 may be connected to the magnetic actuation device 100 and the imaging device 300 to respectively control these devices. The control device may include a computer device or the like, and for example, the imaging device 300 and the control device 400 may be configured as one device.

The imaging device 300 and the control device 400 may each be a general medical device used in a hospital, and are not limited as long as these devices may be connected to the magnetic actuation device 100.

The magnetic field generation module 120 may further include a sensor unit 125 measuring an alignment direction of the permanent magnets 10 set by the rotations of the permanent magnets 10. The sensor unit 125 may measure the alignment direction of the permanent magnets 10 set by the rotations of the permanent magnets 10 about the vertical axis Cz or the horizontal axis Cx, and transmit a measured value to the control device 400. The control device 400 may transmit an actuation signal to the actuation unit 123 of the magnetic field generation module 120 to control the set alignment direction. The sensor unit 125 may be a hall sensor.

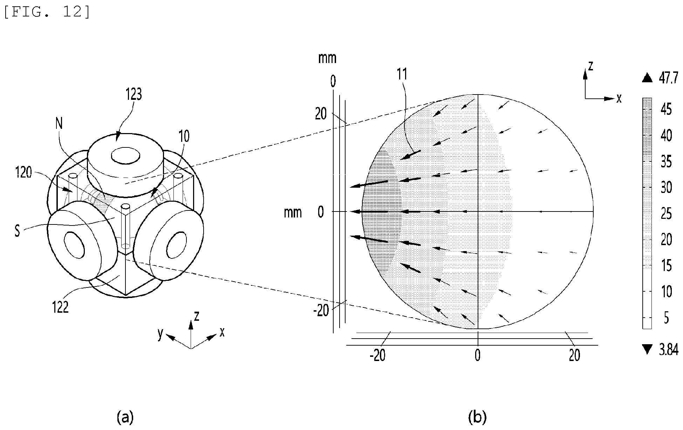

FIG. 12 shows distribution of the magnetic field generated by the electromagnetic coil 123a to control the alignment direction of the permanent magnets 10. The electromagnetic coil 123a may control the distribution of the magnetic field. The control of the electromagnetic coil 123a may be for alignment of the permanent magnets 10 in a desired direction. When the actuation unit 123 uses the electromagnetic coil 123a, the alignment direction of the permanent magnets 10 may be controlled by controlling the distribution of the magnetic field by the electromagnetic coil 123a (here, red is an N pole, and blue is an S pole).

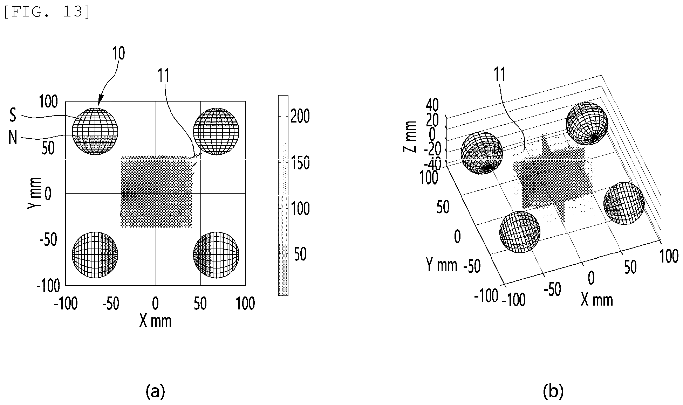

FIGS. 13A and 13B are views each showing the distribution of the magnetic field measured by disposing the plurality of magnetic actuation devices 100. It may be seen that directions 11 of the magnetic field may be aligned as shown in FIGS. 13A and 13B when using the plurality of magnetic actuation devices 100 and controlling the alignment direction of the permanent magnets 10 in each magnetic actuation device 100. The directions 11 of the magnetic field may be aligned in the desired direction not only on a two-dimensional plane as shown in FIG. 13A but also in three dimensions as shown in FIG. 13B.

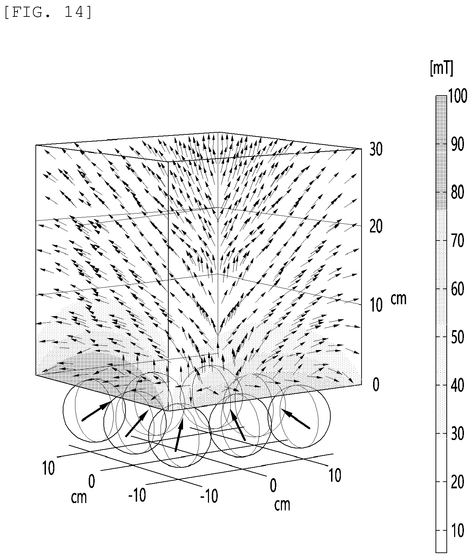

In addition, FIG. 14 is a view showing that the distributions of the magnetic field are aligned in the magnetic actuation device 100 installed in the magnetic actuation system 200 of FIG. 10. It may be seen that the directions 11 of the magnetic field are aligned on an upper side of the bed 210 where the patient 1 lies down through the alignment of the permanent magnets 10.

In addition, as shown in FIGS. 12 to 14, it is possible to adjust not only the directions 11 of the magnetic field but also the strength of the magnetic field by aligning the magnetic actuation device 100 (the darker a color, the stronger the magnetic force in a bar graph of each drawing).

In the embodiments of the present disclosure described above, the magnetic actuation system may control a magnetic actuation-type robot used as the medical device by controlling the direction of the two or more permanent magnets 10 set by the respective rotations of the permanent magnets 10 in two axial directions Cxz. The magnetic actuation system may control the magnetic-actuation type robot by applying the three-dimensional magnetic field and magnetic force to the magnetic actuation-type robot or the like by controlling the direction of the permanent magnet 10. That is, the magnetic actuation system may apply the magnetic field and the magnetic force to the magnetic actuation-type robot in the desired direction by controlling the alignment direction of each permanent magnet 10.

As set forth above, an embodiment of the present disclosure may provide the magnetic actuation device using the permanent magnet which may generate the magnetic field for a long time with the lower power and lower heat by using the permanent magnet, and the system using the same.

In addition, the magnetic actuation device using the permanent magnet may utilize the plurality of permanent magnets for controlling an alignment direction with two degrees of freedom to thus exclude an actuation element limiting space utilization, such as a robot arm, thus allowing an operator using the medical device to perform a safe procedure on the patient.

In addition, the present disclosure may provide the magnetic actuation device using the permanent magnet which may be easily used together with a medical imaging system such as an X-ray system by having an open working space with the permanent magnet having a small volume, and the system using the same.

In addition, the present disclosure may provide the magnetic actuation device using the permanent magnet in which the plurality of permanent magnets used to generate the magnetic field may control the alignment direction with the two degrees of freedom to thus generate the magnetic field with the higher degree of freedom, and the system using the same.

## Claims

1. A magnetic actuation device comprising:
a base; and
at least two magnetic field generation modules each disposed on the base and including a permanent magnet, the permanent magnet being rotated about a horizontal axis parallel to the base or a vertical axis perpendicular to the base.

2. The device of claim 1, wherein the magnetic field generation module includes
a rotation unit rotating the permanent magnet in two axial directions of the horizontal axis or the vertical axis,
a fixing unit fixing the rotation unit, and
an actuation unit transmitting a rotational force to the rotation unit.

3. The device of claim 1 or 2, wherein the fixing unit has an accommodation space for accommodating the permanent magnet, and
the rotation unit is formed in the fixing unit as a rotation groove accommodating a guide ball guiding the rotation of the permanent magnet.

4. The device of any of the preceding claims, wherein the actuation unit is an electromagnetic coil controlling a rotation direction of the permanent magnet based on a current, and is disposed on an outer surface of the rotation unit.

5. The device of any of the preceding claims, wherein the rotation unit includes
a first rotation ring installed on the fixing unit and rotating the permanent magnet about the vertical axis, and
a second rotation ring installed on the first rotation ring and rotating the permanent magnet about the horizontal axis.

6. The device of any of the preceding claims, wherein the actuation unit is an actuator, and is connected to each of the first rotation ring and the second rotation ring to transmit the rotational force in each direction.

7. A magnetic actuation system which includes the magnetic actuation device according to any one of claims 1 to 6, the system comprising:
a bed;
the magnetic actuation device installed in the bed;
an imaging device connected to the magnetic actuation device; and
a control device controlling the magnetic actuation device and the imaging device.

8. The system of claim 7, wherein the magnetic actuation device is installed in a rear surface of the bed or in a support part fixed to the bed.

9. The system of claim 7 or 8, wherein the magnetic field generation module further includes a sensor unit measuring an alignment direction of the permanent magnets set by the rotations of the permanent magnets.
